(19) Europäisches Patentamt / European Patent Office / Office européen des brevets

(11) **EP 2 736 559 B1**

(12) **EUROPÄISCHE PATENTSCHRIFT**

(45) Veröffentlichungstag und Bekanntmachung des Hinweises auf die Patenterteilung:
**24.05.2017 Patentblatt 2017/21**

(51) Int Cl.:
**A61M 1/36** *(2006.01)*

(21) Anmeldenummer: **12737205.0**

(22) Anmeldetag: **10.07.2012**

(86) Internationale Anmeldenummer:
**PCT/EP2012/002908**

(87) Internationale Veröffentlichungsnummer:
**WO 2013/017194 (07.02.2013 Gazette 2013/06)**

(54) **VERFAHREN ZUM ERMITTELN WENIGSTENS EINES PARAMETERS EINES EXTRAKORPORALEN BLUTKREISLAUFS SOWIE VORRICHTUNGEN**

METHOD FOR DETERMINING AT LEAST ONE PARAMETER OF AN EXTRACORPOREAL BLOOD CIRCUIT, AND DEVICES

PROCÉDÉ DE DÉTERMINATION D'AU MOINS UN PARAMÈTRE D'UN CIRCUIT SANGUIN EXTRACORPOREL ET DISPOSITIFS CORRESPONDANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2011 DE 102011108786**
**29.07.2011 US 201161513005 P**

(43) Veröffentlichungstag der Anmeldung:
**04.06.2014 Patentblatt 2014/23**

(73) Patentinhaber: **Fresenius Medical Care Deutschland GmbH**
**61352 Bad Homburg (DE)**

(72) Erfinder:
• **NOACK, Joachim**
**97616 Bad Neustadt (DE)**
• **ZHANG, Wei**
**97464 Niederwerrn (DE)**

(74) Vertreter: **Bobbert & Partner Patentanwälte PartmbB**
**Postfach 1252**
**85422 Erding (DE)**

(56) Entgegenhaltungen:
DE-A1-102008 045 422    DE-A1-102009 024 468
US-A1- 2010 192 686    US-B1- 6 270 673
US-B2- 7 947 180

EP 2 736 559 B1

**Beschreibung**

Die vorliegende Erfindung betrifft ein Verfahren gemäß Anspruch 1 zum Ermitteln eines Füllvolumens eines extrakorporalen Blutkreislaufs. Sie betrifft ferner eine Steuereinrichtung gemäß Anspruch 4 sowie eine Behandlungsvorrichtung gemäß Anspruch 5. Des Weiteren betrifft die vorliegende Erfindung ein digitales Speichermedium gemäß Anspruch 7 sowie ein Computerprogramm-Produkt gemäß Anspruch 8. Extrakorporale Blutkreisläufe erfahren in der Praxis vor ihrer Verwendung in einem medizinischen Behandlungsverfahren eine Vorbereitung. Die zur Behandlung mit dem extrakorporalen Blutkreislauf eingesetzte Behandlungsvorrichtung wird in der Praxis vor Behandlungsbeginn an die anstehende Behandlungsmodalität und/oder an die verwendeten (Einweg-)Artikel wie Blutkreislauf oder Blutfilter angepasst. Zu diesem Zweck wird der extrakorporale Blutkreislauf üblicherweise vor seinem Gebrauch mit einem Fluid (beispielsweise einer Substituatlösung) befüllt und hiermit gespült um möglicherweise vorhandene Produktionsrückstände und/oder Luftblasen aus dem extrakorporalen Blutkreislauf zu entfernen; an der Behandlungsvorrichtung wird gelegentlich eingestellt, mit welcher Behandlungsmodalität behandelt werden soll oder darf, oder von welchem Typ oder von welcher Größe der eingesetzte Blutfilter oder Blutkreislauf ist. Sowohl zum Bestimmen eines Mindestspülvolumens für den Blutkreislauf als auch für das Einstellen der Behandlungsvorrichtung auf die verwendeten (Einweg-)Artikel ist es erforderlich, wenigstens einen Parameter hierüber zu kennen.DE 10 2008 045 422 A1 (4.3.2010) offenbart ein Verfahren zum Berechnen eines Füllvolumens eines extrakorporalen Blutkreislaufs. Eine Aufgabe der vorliegenden Erfindung ist es, ein Verfahren zum, vorzugsweise automatischen, Ermitteln wenigstens eines Parameters eines extrakorporalen Blutkreislaufs anzugeben. Ferner sollen eine zur Durchführung des erfindungsgemäßen Verfahrens vorgesehene Steuereinrichtung, eine Behandlungsvorrichtung, mit welcher das erfindungsgemäße Verfahren durchführbar ist, sowie ein geeignetes digitales Speichermedium, ein geeignetes Computerprogramm-Produkt und ein geeignetes Computerprogramm angegeben werden.

Die erfindungsgemäße Aufgabe wird durch ein Verfahren mit den Merkmalen des Anspruchs 1 gelöst. Sie wird zudem durch eine Steuereinrichtung mit den Merkmalen des Anspruchs 20 sowie eine Behandlungsvorrichtung mit den Merkmalen des Anspruchs 21 gelöst. Die erfindungsgemäße Aufgabe wird ferner gelöst durch ein digitales Speichermedium, ein Computerprogramm-Produkt sowie ein Computerprogramm gemäß den Ansprüchen 23, 24 und 25.

Alle mit dem erfindungsgemäßen Verfahren erzielbaren Vorteile lassen sich in bestimmten erfindungsgemäßen Ausführungsformen ungeschmälert auch mit den erfindungsgemäßen Vorrichtungen erzielen.

Das erfindungsgemäße Verfahren ist geeignet und vorgesehen zum Ermitteln wenigstens eines Parameters eines extrakorporalen Blutkreislaufs.

Das erfindungsgemäße Verfahren umfasst das Befüllen des extrakorporalen Blutkreislaufs durch Einbringen eines medizinischen Fluids.

Es umfasst ferner das Erfassen oder Bestimmen der Größe eines Volumens oder einer Menge des eingebrachten Fluids, welches zum Befüllen des extrakorporalen Blutkreislaufs oder zum Befüllen eines bestimmten Abschnitts hiervon (beispielsweise bis zu einem Detektorabschnitt oder bis an eine Erkennungseinrichtung wie einen Luftdetektor heran) erforderlich ist.

Das Erfassen oder Bestimmen des Volumens oder der Menge erfolgt mittels einer Einrichtung, insbesondere automatisch.

Die erfindungsgemäße Steuereinrichtung, welche auch eine Erfassungseinrichtung sein kann oder als solche einsetzbar sein kann, ist geeignet und vorgesehen und/oder ausgelegt und/oder konfiguriert zur Durchführung des erfindungsgemäßen Verfahrens.

Die erfindungsgemäße Behandlungsvorrichtung ist vorgesehen und ausgestaltet und/oder ausgestattet und/oder konfiguriert zur Durchführung des erfindungsgemäßen Verfahrens und/oder zu ihrer automatischen Einstellung basierend auf Ergebnissen des erfindungsgemäßen Verfahrens.

Dabei können alle, einige oder manche der maschinell durchgeführten Schritte des erfindungsgemäßen Verfahrens veranlasst werden.

Ein erfindungsgemäßes Computerprogramm-Produkt weist einen auf einem maschinenlesbaren Träger gespeicherten Programmcode zur Veranlassung der maschinellen Schritte eines erfindungsgemäßen Verfahrens, wenn das Computerprogramm-Produkt auf einem Computer abläuft, auf.

Ein "maschinenlesbarer Träger" bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein erfindungsgemäßes Computerprogramm weist einen Programmcode auf zur Veranlassung der maschinellen Schritte eines erfindungsgemäßen Verfahrens, wenn das Computerprogramm auf einem Computer abläuft.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden.

Ein erfindungsgemäßes, insbesondere digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart mit einem programmierbaren Computer oder Computersystem zusammenwirken, dass die maschinellen Schritte eines hierin beschriebenen erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen auf einem maschinenlesbaren Träger oder Speichermedium gespeicherten Programmcode auf zur Veranlassung der Schritte des erfindungsgemäßen Verfahrens, wenn das Computerprogramm-Produkt auf einem Rechner abläuft. Unter einem Computerprogramm-Produkt kann erfindungsgemäß beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Ein erfindungsgemäßes Computerprogramm weist einen Programmcode auf zur Veranlassung der maschinellen Schritte eines erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Erfindungsgemäße Ausführungsformen können manche, einige oder alle der folgenden Merkmale in beliebiger Kombination aufweisen. Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Ein extrakorporaler Blutkreislauf weist in bestimmten Ausführungsformen der vorliegenden Erfindung eine medizinische Funktionseinrichtung auf. Diese Funktionseinrichtung kann beispielsweise eine (insbesondere als Disposable ausgestaltete) Blutkassette, und/oder eine Behandlungseinrichtung, wie beispielsweise einen Dialysator oder einen Blutfilter, und/oder einen Blutschlauchsatz aufweisen oder hieraus bestehen.

Die Größe des Volumens (auch kurz: das Volumen) ist in manchen erfindungsgemäßen Ausführungsformen ein Zahlenwert mit einer Dimension (wie ml; Milliliter) oder ohne Dimension. In anderen erfindungsgemäßen Ausführungsformen wird hierunter eine Klassifizierung des Volumens oder seine Zuordnung zu bestimmten Volumenklassen verstanden.

Das "Befüllen" bezeichnet in bestimmten Ausführungsformen der vorliegenden Erfindung das Einbringen oder Einfüllen von - insbesondere medizinischem - Fluid, z. B. Substituat-und/oder Dialysierflüssigkeit, in den extrakorporalen Blutkreislauf. Das Befüllen erfolgt in einigen erfindungsgemäßen Ausführungsformen einmalig und/oder initial, d. h. vor Beginn der Behandlung des Patienten, beispielsweise eines Blutbehandlungsverfahrens, z. B. einer Dialyse (Hämodialyse, Hämofiltration, Hämodiafiltration oder dergleichen) und/oder vor Beginn des Spülvorgangs.

In bestimmten Ausführungsformen der vorliegenden Erfindung ist das Befüllen im Sinne der vorliegenden Erfindung vor Beginn des Spülvorgangs begonnen, erfolgt oder abgeschlossen.

Der extrakorporale Blutkreislauf wird in manchen erfindungsgemäßen Ausführungsformen beim Befüllen vollständig oder im Wesentlichen vollständig mit Fluid befüllt.

Dabei wird in einigen erfindungsgemäßen Ausführungsformen so viel Fluid in den extrakorporalen Blutkreislauf eingebracht, dass das eingebrachte Volumen einem Innenvolumen oder einem maximalen (Aufnahme-)Volumen des extrakorporalen Blutkreislaufs (mit oder ohne hiermit in Fluidverbindung stehenden Komponenten wie einem Blutfilter oder einer Blutkassette) entspricht.

Um das Volumen des extrakorporalen Blutkreislaufs oder eines Abschnitts hiervon zu erfassen, muss der extrakorporale Blutkreislauf in manchen erfindungsgemäßen Ausführungsformen nicht vollständig mit Fluid befüllt werden.

So wird in bestimmten Ausführungsformen der vorliegenden Erfindung der extrakorporale Blutkreislauf zum Beispiel nur teilweise, beispielsweise bis zu einem bestimmten Punkt, z. B. einer im oder am extrakorporalen Blutkreislauf angeordneten Erkennungseinrichtung, eingesetzt zum Erkennen eines Fluids oder zum Erkennen eines Wechsels zwischen unterschiedlichen Fluiden, mit Fluid befüllt.

In manchen Ausführungsformen kann beispielsweise vorgesehen sein, das (Füll-)Volumen oder Gesamtvolumen oder Gesamtinnenvolumen oder Fassungsvermögen des extrakorporalen Blutkreislaufs hochzurechnen oder abzuschätzen. Diese Hochrechnung oder Abschätzung stützt sich auf die Größe des erfassten Volumens (beispielsweise ist dies der befüllte Abschnitt des extrakorporalen Blutkreislaufs zwischen dem Einbringungsort des Fluids und dem Erkennungsort) und auf die Kenntnis des verbleibenden Teils des gesamten extrakorporalen Blutkreislaufs.

Das in den extrakorporalen Blutkreislauf eingebrachte Fluid ist in manchen erfindungsgemäßen Ausführungsformen eine Priming- und/oder Spülflüssigkeit, beispielsweise eine Substituatflüssigkeit.

In bestimmten Ausführungsformen umfasst das erfindungsgemäße Verfahren das Weiterverarbeiten der erfassten Größe des Volumens des Fluids. Das Weiterverarbeiten dient in manchen erfindungsgemäßen Ausführungsformen zum Erhalten des wenigstens einen Parameters oder ganz allgemein einer Angabe.

Das Weiterverarbeiten der erfassten Größe ist oder umfasst in einigen erfindungsgemäßen Ausführungsformen ein Treffen einer Aussage über den Parameter.

Das Weiterverarbeiten der erfassten Größe des Volumens ist oder umfasst in einigen erfindungsgemäßen Ausführungsformen ein Umrechnen des Wertes des Volumens über Algorithmen, Funktionen oder Zusammenhänge zum Erhalt des Parameters. Sie können in einer Speichereinrichtung, z. B. einer Speicher- oder Steuereinrichtung einer Behandlungsvorrichtung, hinterlegt sein oder werden.

Das Weiterverarbeiten der erfassten Größe des Volumens ist oder umfasst in manchen erfindungsgemäßen Ausführungsformen ein Mitteilen des erhaltenen Parameters an eine Aufsichtsperson, in anderen Ausführungsformen hingegen nicht.

Das Weiterverarbeiten der erfassten Größe des Volumens ist oder umfasst in bestimmten erfindungsgemäßen Ausführungsformen ein Vergleichen des erfassten Volumens mit zur Ermittlung des Parameters geeigneten Größen oder Angaben (beispielsweise anhand einer Vergleichstabelle).

Das Weiterverarbeiten der erfassten Größe des Volumens ist oder umfasst in gewissen erfindungsgemäßen Ausführungsformen ein Zuordnen eines Parameters zum extrakorporalen Blutkreislauf und/oder zu einzelnen Komponenten hiervon basierend auf der erfassten Größe des Volumens.

Das Weiterverarbeiten der erfassten Größe des Volumen umfasst in manchen erfindungsgemäßen Ausführungsformen ein Mitteilen des Parameters an eine Aufsichtsperson (beispielsweise mittels Displayanzeige, Fehlermeldung, Alarm oder dergleichen), in anderen nicht.

Ein Parameter ist in einigen erfindungsgemäßen Ausführungsformen eine Angabe, welche charakteristisch für den verwendeten extrakorporalen Blutkreislauf ist. Hierzu zählen Angaben, welche den extrakorporalen Blutkreislauf beschreiben, ihn unterscheidbar machen, seine Größe angeben, seine Typenzugehörigkeit, seine Eignung für bestimmte Behandlungsmodalitäten oder -verfahren oder seine Eignung zur Verwendung mit bestimmten Behandlungsvorrichtungen angeben, Aussagen über einzelne Komponenten hiervon machen (etwa: vorhanden oder nicht vorhanden), und dergleichen.

Ein Parameter im Sinne der vorliegenden Erfindung kann ein Parameterwert, eine Ausprägung oder eine Größe eines Parameters, ein Zahlenwert mit Dimension, ein Zahlenwert ohne Dimension, eine Typzugehörigkeit, eine Klassenzugehörigkeit und dergleichen sein.

Ein Parameter ist in gewissen erfindungsgemäßen Ausführungsformen eine Angabe, welche charakteristisch für den verwendeten extrakorporalen Blutkreislauf ist, und zwar alternativ ausschließlich (die Angabe oder der Parameter ist eindeutig nur dem verwendeten extrakorporalen Blutkreislauf oder dem verwendeten Typ von extrakorporalen Blutkreisläufen zugeordnet) oder nicht ausschließlich (die Angabe oder der Parameter ist neben dem verwendeten extrakorporalen Blutkreislauf oder dem verwendeten Typ von extrakorporalen Blutkreisläufen auch weiteren Blutkreisläufen oder Typen von extrakorporalen Blutkreisläufen zugeordnet, lässt aber dennoch eine gewisse Zuordnung, etwa zu einer abgeschlossenen Gruppe von extrakorporalen Blutkreisläufen, zu).

Das Volumen des Fluids, welches zum Befüllen des extrakorporalen Blutkreislaufs in diesen eingebracht wird, ist in bestimmten Ausführungsformen der vorliegenden Erfindung ein kumuliertes Volumen.

Ein kumuliertes Volumen bezeichnet in bestimmten Ausführungsformen der vorliegenden Erfindung das gesamte zum erstmaligen oder einmaligen, insbesondere zum vollständigen oder im Wesentlichen vollständigen, Befüllen des extrakorporalen Blutkreislaufs eingebrachte Volumen an Fluid. Das kumulierte Volumen umfasst also auch Teilvolumina, welche zum Erzielen des erstmaligen Befüllens des extrakorporalen Blutkreislaufs z. B. aufgrund einer während und aufgrund des Befüllens erfolgenden Entlüftung des extrakorporalen Blutkreislaufs zum Erzielen des angestrebten Befüllens ersetzt oder nachgefüllt werden müssen.

Das Erreichen des befüllten Zustands des extrakorporalen Blutkreislaufs wird mittels einer Erkennungseinrichtung zum Erkennen eines Fluids oder eines Fluidzustands (wie einer Färbung, eines Mischungsverhältnisses, eines Wechsels unterschiedlicher Fluide, etwa eines Verdrängens von Luft durch das Fluid, einer Grenze zwischen einem flüssigen Fluid und Luft, usw.) festgestellt.

Die Erkennungseinrichtung ist in gewissen Ausführungsformen der vorliegenden Erfindung im oder am extrakorporalen Blutkreislaufs angeordnet. In einigen erfindungsgemäßen Ausführungsformen steht sie mit diesem in Wirk- oder Signalverbindung.

In bestimmten Ausführungsformen der vorliegenden Erfindung ist die Erkennungseinrichtung ausgewählt aus Pegeldetektoren (beispielsweise Ultraschall-Pegeldetektoren, kapazitiven Pegeldetektoren, optischen Pegeldetektoren oder dergleichen), Luftdetektoren (beispielsweise Luftblasendetektoren), venösen Blasenfängern, oder dergleichen, oder Kombinationen hiervon, welche nach demselben physikalischen Prinzip oder nach unterschiedlichen Prinzipien arbeiten.

In manchen Ausführungsformen der vorliegenden Erfindung wird eine Laufzeit eines mittels einer Blutpumpe in extrakorporalen Blutkreislauf geförderten Fluids (welches in diesen Ausführungsformen beispielsweise Dialysierflüssigkeit, Substituatflüssigkeit oder Blut ist) bestimmt oder errechnet. In diesen Ausführungsformen umfasst das erfindungsgemäße Verfahren das Fördern des Fluids durch den extrakorporalen Blutkreislauf oder Abschnitte hiervon mittels einer Pumpe, beispielsweise der Blutpumpe. Diese fördert entsprechend eines gewünschten konstanten Flusses oder eines nichtkonstanten aber auf andere Weise vorbestimmten Flussverlaufs, welcher eine Berechnung des innerhalb einer betrachteten Zeitspanne geförderten Fluidvolumens erlaubt. Ein solcher vorbestimmter Fluss kann beispielsweise ein sich kontinuierlich verstärkender Fluss oder ein sich zu vorbestimmten Zeitpunkten sprungweise erhöhender Fluss bei bekannter Sprunghöhe sein.

In diesen Ausführungsformen wird der Zeitpunkt des Auftretens des Fluids an einem ersten Sensor des extrakorporalen Blutkreislaufs ermittelt. Dieser Zeitpunkt wird als erster Zeitpunkt bezeichnet.

In diesen Ausführungsformen wird der Zeitpunkt des Auftretens des Fluids an einem zweiten Sensor des extrakorporalen Blutkreislaufs ermittelt. Dieser Zeitpunkt wird als zweiter Zeitpunkt bezeichnet.

In diesen Ausführungsformen wird die Zeitdifferenz zwischen dem ersten Zeitpunkt und dem zweiten Zeitpunkt ermittelt.

In diesen Ausführungsformen ist das Erfassen, Ermitteln oder Berechnen der Größe des Volumens des eingebrachten Fluids eine Multiplikation der Zeitdifferenz mit der konstanten Flussrate der Blutpumpe oder umfasst eine solche Multiplikation.

In diesen Ausführungsformen kann das Volumen des extrakorporalen Blutkreislaufs somit anhand der Laufzeit des Fluids, beispielsweise des Bluts, vom ersten Sensor zum zweiten Sensor ermittelt werden, wenn die Förderleistung oder -menge der verwendeten Pumpe, hier nur beispielsweise einer Blutpumpe, bekannt ist. Die Förderleistung oder -menge der Pumpe kann sich aus einer als konstant oder anderweitig bekannten Förderrate der Pumpe zwischen dem ersten und dem zweiten Zeitpunkt ergeben.

Auf diese Weise lässt sich anhand der Zeitdifferenz oder der Laufzeit und der Pumpenrate, - leistung oder der -menge pro Zeit das Gesamtvolumen des geförderten Fluids und damit das von den Schlauchabschnitten und der Behandlungseinrichtung (z. B. einem Dialysator) gemeinsam aufgenommene Füllvolumen bestimmen.

Ist das Füllvolumen des extrakorporalen Blutkreislaufs, insbesondere der Schlauchabschnitte hiervon, bekannt, so lässt sich der verwendete Typ der Behandlungseinrichtung (z. B. des Dialysators) bestimmen, da jeder Typ einer Behandlungseinrichtung ein für ihn typisches Volumen aufnimmt.

Jeder der hierin genannten optischen Sensoren kann ein venöser Blutdetektor (zum Detektieren von Blut im venösen Schenkel des extrakorporalen Blutkreislaufs) auf optischer Basis, ein optischer Sensor zur Messung wenigstens eines Blutparameters, oder jeder andere Typ eines Sensors sein. Unter einem solchen Blutparameter sind u. a. das relative Blutvolumen, die Hämoglobinkonzentration und der Hämatokrit zu verstehen. Andere optische Detektoren, welche ebenfalls während des erfindungsgemäßen Verfahrens verwendet werden können, sind nicht zum Messen von Blutparametern ausgestaltet.

Ein wie vorstehend beschriebener optischer Sensor zur Messung wenigstens eines Blutparameters ist in gewissen Ausführungsformen der vorliegenden Erfindung eine Vorrichtung für Messungen im Blut wie sie in der Offenlegungsschrift EP 1 748 292 A1 und/oder in der Offenlegungsschrift WO 2004/057313 A1 offenbart ist.

Ein optischer Sensor zur Messung wenigstens eines Blutparameters kann insbesondere eine Vorrichtung sein, welche eine Messeinheit aufweist, die einen Lichtemitter und einen Lichtdetektor aufweist. Hiermit wird Licht mit einer vorgegebenen Wellenlänge in einen Abschnitt des extrakorporalen Blutkreislaufs eingebracht, beispielsweise in den arteriellen Abschnitt. Der Abschnitt ist für Licht, insbesondere Infrarotlicht, durchlässig. Mit dem Lichtdetektor wird aus dem Abschnitt austretendes Licht empfangen. Mittels des optischen Sensors zur Messung wenigstens eines Blutparameters kann das Vorliegen von Fluid, beispielsweise Blut, im Abschnitt erkannt werden. Dies kann beispielsweise anhand des Verhältnisses zwischen der Intensität des ein- und der Intensität des austretenden Lichts erfolgen.

In einigen erfindungsgemäßen Ausführungsformen ist der optische Sensor zur Messung wenigstens eines Blutparameters ausgestaltet, um weitere Blutparameter wie relatives Blutvolumen, Hämoglobinkonzentration, Hämatokrit zu messen. Die Steuereinrichtung ist entsprechend konfiguriert oder eingerichtet.

Wird die Anwesenheit von Fluid mittels Licht festgestellt, so ist in bestimmten erfindungsgemäßen Ausführungsformen die Verwendung von Licht mit einer Wellenlänge von 790 bis 820 nm, bevorzugt von 800 bis 810 nm und besonders bevorzugt von 805 nm vorgesehen.

Soll die die Anwesenheit von Fluid mittels Licht festgestellt werden, so sind in manchen erfindungsgemäßen Ausführungsformen der Lichtemitter und der Lichtdetektor an einem Abschnitt des extrakorporalen Blutkreislaufs für eine Messung in diesem Abschnitt oder durch diesen hindurch angeordnet, welcher zwischen der arteriellen Patientennadel und der Blutpumpe liegt.

Dieser Abschnitt ist in gewissen erfindungsgemäßen Ausführungsformen steifer (hierin auch als stabiler geführt oder aufgenommen bezeichnet) als wenigstens ein anderer Abschnitt des extrakorporalen Blutkreislaufs, als ein stromabwärts der Blutpumpe angeordneter Abschnitt, oder als alle anderen Abschnitte des extrakorporalen Blutkreislaufs.

Der steifere Abschnitt ist in manchen erfindungsgemäßen Ausführungsformen ein Abschnitt, welcher zumindest teilweise an seinem Äußeren oder Umfang von einer Begrenzung oder Manschette (im Folgenden kurz: Manschette) umgeben ist. Die Manschette übt in gewissen erfindungsgemäßen Ausführungsformen Druck auf den durch sie hindurch geführten Abschnitt aus, beispielsweise derart, dass der Abschnitt im Inneren der Manschette ein anderes Querschnittprofil aufweist als vor und/oder hinter der Manschette. Aus diesem Grund kann er als steif(er) bezeichnet werden. Dies kann die Messgenauigkeit mittels Lichtdetektor vorteilhaft erhöhen.

Beispielsweise kann der an sich runde oder kreisförmige Querschnitt des Blutschlauchs in der Manschette in einen eher eckigen oder viereckigen Querschnitt oder einen Querschnitt mit wenigstens einer oder zwei geraden Seitenflächen geformt werden. Ein Beispiel einer solchen Manschette oder der ihr zugrunde liegenden Idee ist in den oben genannten Offenlegungsschriften EP 1 748 292 A1 und WO 2004/057313 A1 offenbart, siehe beispielsweise jeweils die Fig. 1 bis 5 dort.

Insbesondere kann die Pressung des Blutschlauchs durch die Manschette einen vorbestimmten Druck und/oder eine vorbestimmte Form- oder Querschnittsveränderung bewirken. Der Blutschlauchabschnitt kann somit auf definierte Weise verpresst oder gepresst sein.

Insbesondere kann die Manschette in gewissen erfindungsgemäßen Ausführungsformen ausgestaltet sein als ein längliches Behältnis.

Insbesondere kann die Manschette in gewissen erfindungsgemäßen Ausführungsformen ausgestaltet sein mit Aufnahmen oder Durchlässe für Lichtemitter und/oder Lichtdetektor.

Sowohl der erste Sensor als auch der zweite Sensor können unabhängig voneinander als Sensoren ausgestaltet sein, welche mittels Messung der optischen Dichte, Ultraschallmessung, Akustikmessung oder Temperaturmessung die Anwesenheit von Fluid feststellen.

In einigen Ausführungsformen der vorliegenden Erfindung wird die ermittelte Zeitdifferenz zu Zwecken der Verfahrenssteuerung der Blutbehandlung oder des Abrüstens des Blutbehandlungsvorrichtung nach abgeschlossener Behandlung genutzt. Beispielsweise kann die Kenntnis der Zeitdifferenz für einen automatischen Stopp der Reinfusion des Blutes nach Behandlungsende verwendet werden.

In gewissen Ausführungsformen der vorliegenden Erfindung ist die Erkennungseinrichtung dazu vorgesehen, eine Fluid-Luft-Grenze zu erkennen. Sie kann somit angeben, wann die im (noch unbenutzten) extrakorporalen Blutkreislauf befindliche Luft stromaufwärts der Erkennungseinrichtung durch das eingefüllte Fluid verdrängt und beispielsweise über eine Entlüftungseinrichtung aus dem extrakorporalen Blutkreislauf entfernt wurde.

In bestimmten Ausführungsformen der vorliegenden Erfindung wird das Volumen des Fluids über die (Anzahl der) Rotordrehungen einer Fördereinrichtung, mittels welcher der extrakorporale Blutkreislauf befüllt wurde, erfasst.

Bei der Fördereinrichtung kann es sich um eine Schlauchrollenpumpe handeln, zum Beispiel eine Schlauchrollenpumpe, welche in Behandlungsverfahren zum Fördern von Blut (Blutpumpe) und/oder Substituat (Substituatpumpe) und/oder Dialysat eingesetzt wird.

In weiteren Ausführungsformen der vorliegenden Erfindung wird das Volumen des Fluids über die Gewichtsabnahme einer Quelle für das Fluid, z. B. eines Flüssigkeitsbeutels, erfasst. Die Gewichtsabnahme kann beispielsweise durch Wiegen des Flüssigkeitsbeutels erfasst werden.

In bestimmten Ausführungsformen der vorliegenden Erfindung umfasst das erfindungsgemäße Verfahren das Vergleichen des ermittelten Parameters mit (erfassten und hinterlegten) Parametern einer Vielzahl einsetzbarer medizinischer Funktionseinrichtungen zur Identifikation oder Zuordnung des extrakorporalen Blutkreislaufs einer hierin vorliegenden oder verwendeten medizinischen Funktionseinrichtung oder Komponente. Ein solches Vergleichen entspricht in manchen erfindungsgemäßen Ausführungsformen dem oben diskutierten Weiterverarbeiten oder umfasst dieses.

Das Vergleichen kann z. B. mittels einer Vergleichstabelle erfolgen.

In weiteren Ausführungsformen umfasst das erfindungsgemäße Verfahren das Vergleichen des ermittelten Parameters mit (erfassten und hinterlegten) Parametern einer Vielzahl einsetzbarer Behandlungseinrichtungen zur Identifikation einer verwendeten Behandlungseinrichtung.

Die Vielzahl der einsetzbaren medizinischen Funktionseinrichtungen oder der einsetzbaren Behandlungseinrichtungen oder Informationen jeweils zu ihnen können in geeigneten Tabellen hinterlegt sein.

Ein vor einer Verwendung des extrakorporalen Blutkreislaufs zu einer Behandlung üblicherweise erfolgendes Vorbereiten, beispielsweise durch Primen und/oder Spülen dient wie oben erwähnt dazu, etwaige Produktionsrückstände (bedingt durch die Produktion der Behandlungseinrichtung und/oder des Blutschlauchsatzes) und/oder Luft aus dem Blutkreislauf oder den hiermit verbundenen Komponenten zu entfernen. Die zum Spülen oder Primen erforderliche Fluidmenge oder das erforderliche Fluidvolumen ist üblicherweise größer als die zum einfachen Befüllen des extrakorporalen Blutkreislaufs erforderliche Fluidmenge bzw. das Fassungs- oder Fluidvolumen. Im Stand der Technik wird zum Primen/Spülen ein allgemein gültiges Mindestvolumen von z. B. 500 ml festgelegt. Diese Festlegung ist offensichtlich nicht auf den jeweils konkret verwendeten extrakorporalen Blutschlauchsatz und seine Komponenten, etwa den verwendeten Blutfilter, abgestimmt, angepasst oder gar optimiert.

Das erfindungsgemäße Verfahren umfasst in bestimmten Ausführungsformen das Festlegen eines minimalen, optimalen und/oder maximalen Priming- oder Spülvolumens zum Primen oder Spülen des extrakorporalen Blutkreislaufs anhand des ermittelten Parameters. Damit wird das Priming- oder Spülvolumen anhand des ermittelten Parameters vorteilhaft auf den tatsächlich verwendeten extrakorporalen Blutkreislauf und seine Komponenten angepasst. Hierbei kann der Parameter beispielsweise das Füllvolumen des Blutkreislaufs oder dessen Typ sein.

In gewissen Ausführungsformen kann ein Mindestspülvolumen V_min zur Vorbereitung des später erfolgenden Behandlungsverfahrens, welches nicht Gegenstand oder Teil der vorliegenden Erfindung ist, beispielsweise der folgenden mathematischen Funktion folgen:

$$V\_min = a*V\_Set + b*V\_Dial$$

Hierbei ist V_min das Mindestspülvolumen bei der Vorbereitung des Behandlungsverfahrens; V_Set ist das Volumen der medizinischen Funktionseinrichtung, insbesondere eines Blutschlauchsatzes oder Blutschlauchsets; V_Dial ist das Volumen der Behandlungseinrichtung, insbesondere eines Dialysators oder Blutfilters; und a und b sind jeweils Zahlen oder Konstanten.

In bestimmten Ausführungsformen ist a eine beliebige Zahl, welche Werte insbesondere von 1 bis 3 annehmen kann.

In gewissen Ausführungsformen ist b eine beliebige Zahl, welche Werte insbesondere von 2 bis 5 annehmen kann.

In manchen Ausführungsformen können in die Bestimmung des Priming- oder Spülvolumens weitere Faktoren, wie beispielsweise (Behandlungs-)Vorschriften oder dergleichen, eingehen.

In bestimmten erfindungsgemäßen Ausführungsformen umfasst das Verfahren das Festlegen oder Beeinflussen einer Steuerung des (späteren) Behandlungsverfahrens unter Einbezug oder in Abhängigkeit des ermittelten Parameters.

In einigen Ausführungsformen umfasst das erfindungsgemäße Verfahren das Festlegen von maximalen und/oder minimalen Pumpenraten, ein (automatisches) Festlegen von Reinfusionsvolumina oder dergleichen unter Einbezug oder in Abhängigkeit des ermittelten Parameters.

In bestimmten erfindungsgemäßen Ausführungsformen sind Reinfusionsvolumina solche Volumina einer Flüssigkeit (z. B. Substituat, Dialysierflüssigkeit oder Spülflüssigkeit), welche erforderlich sind, um das nach Beendigung der Blutbehandlung noch im extrakorporalen Blutkreislauf enthaltene Blut aus diesem herauszuspülen mit dem Ziel, das Blut dem Patienten rückzuführen.

In gewissen Ausführungsformen umfasst das erfindungsgemäße Verfahren das Sperren von Behandlungsmodalitäten und/oder das Einschränken von Behandlungsparametern des (späteren) Behandlungsverfahrens unter Einbezug oder in Abhängigkeit des ermittelten Parameters.

Einige oder alle erfindungsgemäßen Ausführungsformen können einen oder mehrere der oben oder im Folgenden genannten Vorteile aufweisen.

Die vorliegende Erfindung stellt vorteilhaft ein einfaches und wenig aufwendiges Verfahren zur (weiteren) Automatisierung eines Behandlungsverfahrens bereit.

Die vorliegende Erfindung ist vorteilhaft für alle Behandlungsvorrichtungen mit extrakorporalen Blutkreisläufen einsetzbar.

Mittels des erfindungsgemäßen Verfahrens ist es in manchen Ausführungsformen vorteilhaft möglich, basierend auf der beim initialen Befüllen eines extrakorporalen Blutkreislaufs gewonnenen Information des (Füll-)Volumens oder eines anderen Parameters wie des Typs des verwendeten Blutkreislaufs oder dergleichen eine individualisierte Vorgabe des während des nachfolgenden Spülens umzuwälzenden Flüssigkeitsvolumens zu machen. Dies kann zu einer optimalen Spülwirkung, einer Mindestspülwirkung und/oder zu einem sparsamen Verwenden an Spülfluid beitragen.

Mittels des erfindungsgemäßen Verfahrens ist es in einigen Ausführungsformen vorteilhaft möglich, basierend auf der beim initialen Befüllen eines extrakorporalen Blutkreislaufs gewonnenen Information des (Füll-)Volumens gewisse Behandlungsmodalitäten zuzulassen oder zu sperren, entsprechend der erkannten Eignung, Größe, oder des Typs von extrakorporalem Blutkreislauf und/oder verwendeter Behandlungsvorrichtung. Dies kann vorteilhaft dazu beitragen, die Vorbereitung einer Behandlung und/oder den Behandlungsablauf selbst weiter zu automatisieren. Hiermit kann ferner die Sicherheit des Systems sowie die Patientensicherheit erhöht werden.

Die vorliegende Erfindung ist vorteilhaft besonders einfach implementierbar, da sie oft nur ein softwareseitiges Aufrüsten von sich bereits in Betrieb befindenden Behandlungsvorrichtungen umfasst.

Ein derartiges Aufrüsten oder Nachrüsten kann z. B. durch Implementieren des Quell- oder Maschinencodes der Betriebssoftware in der Steuereinrichtung einer Behandlungsvorrichtung erfolgen.

Im Folgenden wird die vorliegende Erfindung unter Bezugnahme auf die beigefügte Zeichnung, in welcher gleiche Bezugszeichen identische oder ähnliche Elemente bezeichnen, rein exemplarisch beschrieben. Es gilt:

Fig. 1 zeigt eine schematische Abbildung eines extrakorporalen Blutkreislaufs, welcher mittels des erfindungsgemäßen Verfahrens zu seiner Verwendung vorbereitet werden kann, sowie eines Dialysatkreislaufs;

**Fig. 2** veranschaulicht das Füllvolumen verschiedener Dialysatoren und deren Identifizierbarkeit mittels des erfindungsgemäßen Verfahrens; und

**Fig. 3** zeigt eine schematische Abbildung eines weiteren extrakorporalen Blutkreislaufs, welcher mittels des erfindungsgemäßen Verfahrens zu seiner Verwendung oder abschließenden Spülung vorbereitet werden kann.

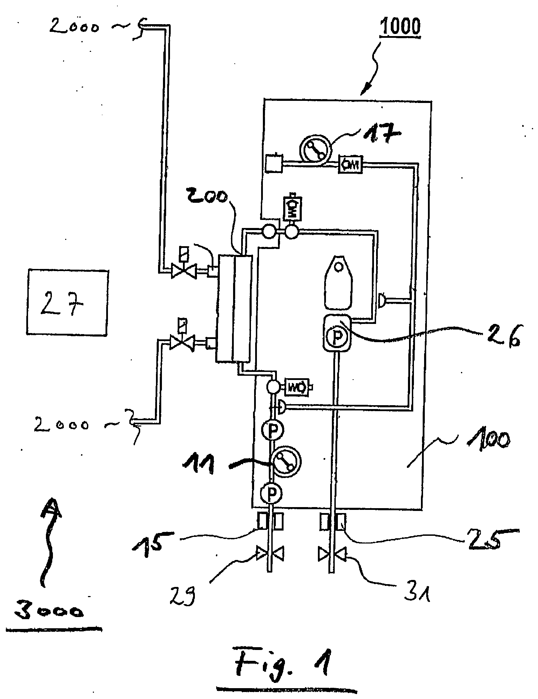

**Fig. 1** zeigt schematisch einen extrakorporalen Blutkreislauf 1000 sowie, andeutungsweise, einen Dialysatkreislauf 2000 einer Behandlungsvorrichtung 3000, beispielsweise einer Hämodiafiltrationsmaschine.

Der extrakorporale Blutkreislauf 1000 weist eine medizinische Funktionseinrichtung 100, z. B. eine (Disposable-)Blutkassette, auf, ist hiermit verbunden oder zumindest abschnittsweise hierin integriert.

Die Funktionseinrichtung 100 ist an die Behandlungsvorrichtung 3000 funktional angekoppelt und wirkt mit den Pumpenantrieben, Aktoren und Sensoren der in Fig. 1 nur angedeuteten Behandlungsvorrichtung 3000 funktional zusammen. Die Pumpenantriebe, Aktoren und Sensoren der Behandlungsvorrichtung 3000 wirken mit einer Steuer- und/oder Regelungseinrichtung 27 der Behandlungsvorrichtung 3000 funktional zusammen. Sie können mit der Steuer- und/oder Regelungseinrichtung in Signalverbindung stehen.

Verbunden ist der extrakorporale Blutkreislauf 1000 mit einer Behandlungseinrichtung 200, beispielsweise einem Dialysator oder einem Blutfilter.

Im oder am extrakorporalen Blutkreislauf 1000 sind eine Blutpumpe 11 sowie eine Substituatpumpe 17 angeordnet. Die Blutpumpe 11 und die Substituatpumpe 17 können in einem später ablaufenden Behandlungsverfahren Blut bzw. Substituat fördern.

Die Blutpumpe 11 und/oder die Substituatpumpe 17 können als Fördereinrichtung(en) zum Fördern des Fluids zum Zwecke des Befüllens des extrakorporalen Blutkreislaufs 1000 im Sinne des erfindungsgemäßen Verfahrens eingesetzt werden. Dasselbe gilt für eine nicht in Fig. 17 dargestellte Pumpe des Dialysatkreislaufs.

Der extrakorporale Blutkreislauf 1000 weist einen arteriellen Luftblasendetektor 15 (auch als "art. ABD" bezeichnet für "arterial air bubble detector") auf. Er weist ferner einen venösen Luftblasendetektor 25 (auch als "ven. ABD" bezeichnet für "venous air bubble detector") auf. Beispielsweise kann der venöse Luftblasendetektor 25 als Erkennungseinrichtung zum Erkennen eines Füllzustandes in den extrakorporalen Blutkreislauf 1000 eingesetzt werden.

Das erfindungsgemäße Verfahren dient in einigen erfindungsgemäßen Ausführungsformen dazu, das Fluidvolumen, welches zum Füllen des extrakorporalen Blutkreislaufs 1000, d. h. der Blutseite des Leitungssystems, benötigt wird, durch die Behandlungsvorrichtung 3000 zu ermitteln.

In bestimmten Ausführungsformen des erfindungsgemäßen Verfahrens wird hierzu das Volumen des zum Befüllen des extrakorporalen Blutkreislaufs 1000 eingebrachten Fluids erfasst, das erforderlich ist, um eine Pegeldetektion (z. B. an einer venösen Kammer 26) oder eine Meldung durch den venösen Luftblasendetektor 25 auszulösen. Das erfasste Volumen kann z. B. durch Ermitteln der benötigten Rotordrehungen der Blutpumpe 11 und/oder der Substituatpumpe 17 oder durch Wägung eines Flüssigkeitsbeutels mit frischer Spül-/Substituatlösung bestimmt werden. Das erfasste Volumen kann mit einem Faktor multipliziert werden, um das benötigte Spülvolumen angeben oder einstellen zu können.

Fällt der Flüssigkeitspegel an der Detektionsstelle, d. h. am Erkennungsort oder an der Erkennungseinrichtung, während des Befüllens wieder ab, z. B. weil sich Restluft aus der Behandlungseinrichtung 200 löst, kann das zum erneuten Anheben des Pegels benötigte Volumen zum bereits ermittelten Volumen hinzuaddiert werden. Das weitere Verwenden des derart erfassten kumulierten Volumens kann dabei vorteilhaft die Genauigkeit der Bestimmung des erfassten Volumens erhöhen.

Das ermittelte Volumen wird in bestimmten Ausführungsformen verwendet zur Identifikation eines verwendeten Blutschlauchsatzes. Die Identifikation des Blutschlauchsatzes kann z. B. anhand einer Vergleichstabelle erfolgen, in der typische Füllvolumina für verschiedene Disposablekonfigurationen oder Blutschlauch/Blutfilter-Kombinationen hinterlegt sind.

Die Vergleichstabelle ist in einigen erfindungsgemäßen Ausführungsformen innerhalb der Steuer- und/oder Regelungseinrichtung 27 in einem Datenspeicher gespeichert oder kann erfindungsgemäß in dieser gespeichert werden.

Die Steuer- und/oder Regelungseinrichtung 27 weist in manchen erfindungsgemäßen Ausführungsformen eine Einrichtung zum Auswerten von Messdaten und Berechnen eines Fördervolumens, insbesondere des kumulierten Fördervolumens aus den Messdaten, auf. Sie kann zusätzlich eine Einrichtung zum Vergleichen des berechneten Fördervolumens mit den Daten aus der Vergleichstabelle aufweisen, zudem eine Einrichtung zum Zuordnen des berechneten Fördervolumens zu bestimmten vorgegebenen Blutschlauchsätzen aus der Vergleichstabelle. Die vorgenannten Einrichtungen können in einer einzigen Einrichtung vereint vorliegen.

So können z. B. vorteilhaft einfach unterschiedliche Blutschlauchsysteme für z. B. pädiatrische und Erwachsenen-Dialyse oder unterschiedliche Behandlungsverfahren (Single-Needle/Double-Needle) unterschieden werden. In Abhängigkeit des identifizierten Disposabletyps können an der Behandlungsvorrichtung, insbesondere automatisch und ohne Zutun der Aufsichtsperson, Behandlungsmodalitäten gesperrt und/oder Behandlungsparameter eingeschränkt werden.

In weiteren Ausführungsformen wird das ermittelte Volumen verwendet zur Identifikation der eingesetzten Behandlungseinrichtung 200, z. B. eines eingesetzten Dialysators.

Zur Verfeinerung der Identifikation können weitere (ergänzende) und der Behandlungsvorrichtung zur Verfügung stehende charakteristische Merkmale hinzugezogen werden (z. B. ein dialysatseitiges Füllvolumen, blut- und/oder dialysatseitige Strömungswiderstände, der Transmembrandruck und dergleichen).

In Fig. 1 sind ferner eine arterielle Blutschlauchklemme 29 sowie eine venöse Blutschlauchklemme 31 dargestellt.

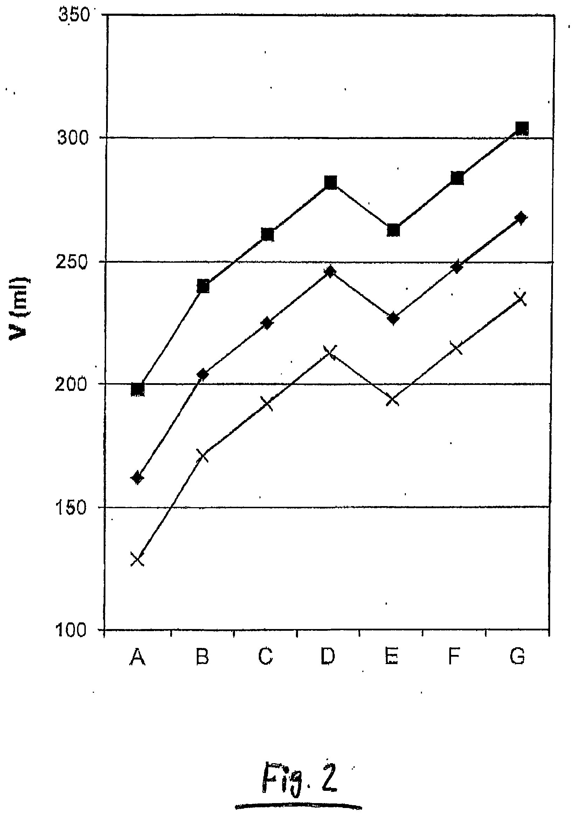

**Fig. 2** zeigt verschiedene Füllvolumina V (in ml) eines ersten extrakorporalen Blutkreislaufs für eine Single-Needle-Behandlung (ihm zugehörige Werte sind mit Quadraten markiert), eines zweiten extrakorporalen Blutkreislaufs für eine Double-Needle-Behandlung (ihm zugehörige Werte sind mit Rauten markiert), und eines dritten extrakorporalen Blutkreislaufs für ein Blutkassette (ihm zugehörige Werte sind mit Kreuzen markiert).

Das Füllvolumen des ersten Blutkreislaufs (Quadrate) beträgt 166 ml. Das Füllvolumen des zweiten Blutkreislaufs (Rauten) beträgt 130 ml. Das Füllvolumen des dritten Blutkreislaufs (Kreuze) beträgt 97 ml. Diese Angaben gelten jeweils für den Blutkreislauf, solange dieser nicht mit einem Blutfilter verbunden ist.

Fig. 2, zeigt, wie sich das Gesamtfüllvolumen der o. g. Blutkreisläufe erhöht, und unterschiedlich erhöht, sobald diese mit einem der Filter A, B, C, D, E, F oder G verbunden sind.

Aus Fig. 2 ist somit gut zu sehen, dass die Kenntnis des Füllvolumens eines konkreten Blutkreislaufs bereits erkennen lässt, mit welchem Filter dieser im Moment seines Befüllens im Sinne der Erfindung verbunden ist.

In bestimmten Ausführungsformen des erfindungsgemäßen Verfahrens umfasst das Verfahren das Festlegen von Parametern oder Parameterwerten zur Verfahrenssteuerung in der nachfolgenden Behandlung, z. B. Festlegung maximal/minimal zulässiger Pumpenraten (kleine Dialysatoren haben kleinere empfohlene Blutflüsse als größere), automatische Festlegung von Reinfusionsvolumina, o. ä.

Tabelle 1, welche dies widerspiegelt, ist im Folgenden für den minimalen Fluss min_Flow und den maximalen Fluss max-Flow für die o. g. Blutfilter A bis G, welche unterschiedliche Fassungs- oder Blutvolumen haben, gezeigt.

Tabelle 1

| Blutfilter | Blutvolumen | min_Flow | max_Flow |
|---|---|---|---|
| A | 32 | 50 | 200 |
| B | 74 | 150 | 400 |
| C | 95 | 200 | 500 |
| D | 116 | 250 | 600 |
| E | 97 | 150 | 400 |
| F | 118 | 200 | 500 |
| G | 138 | 300 | 600 |

Für den Fachmann ist somit erkennbar, dass die Kenntnis des verwendeten Filtertyps, welcher mittels der erfindungsgemäßen Verfahrens ermittelt werden kann, auch für die Steuerung der Behandlungsvorrichtung während einer sich anschließenden Behandlung genutzt werden kann. So kann ein minimaler oder ein maximaler Fluss (Rate) min_Flow oder max_Flow, welcher vom Typen des Blutfilters abhängt, automatisch eingestellt werden.

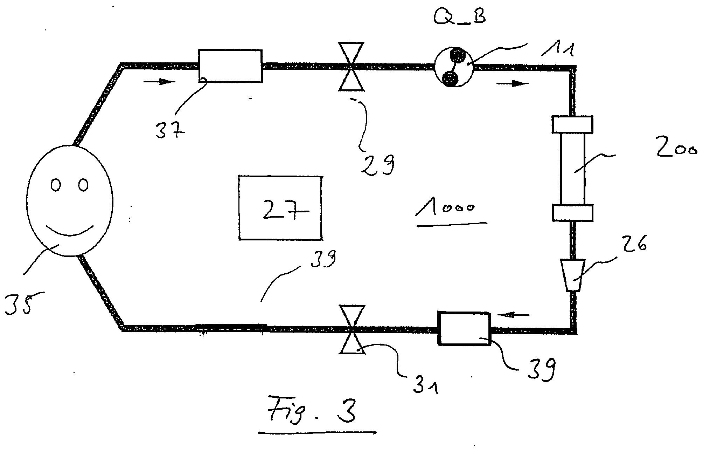

**Fig. 3** zeigt eine schematische Abbildung eines weiteren extrakorporalen Blutkreislaufs 1000. Das erfindungsgemäße Verfahren kann am extrakorporalen Blutkreislaufs 1000 beispielsweise wie folgt ausgeführt werden. Dabei wird angenommen, dass die Blutreinigungseinrichtung 200 ein Dialysator ist:

Nachdem ein sehr schematisch dargestellter und mit dem Bezugszeichen 35 bezeichneter Patient mit dem extrakorporalen Blutkreislauf 1000 konnektiert ist, läuft die Blutpumpe 11 mit einem konstanten Blutfluss $Q\_B$ (ml/min) an. Das Blut strömt an einem ersten Sensor 37 vorbei über die arterielle Blutschlauchklemme 29 des arteriellen Schenkels in den extrakorporalen Blutkreislauf 1000 ein.

Das einströmende Blut wird dabei vom ersten Sensor 37 (beispielsweise einem optischer Sensor, beispielsweise einem optischen Sensor zur Messung wenigstens eines Blutparameters) zu einem ersten Zeitpunkt t1 (sec) erkannt.

Zu einem zweiten Zeitpunkt t2 (sec) wird das Blut im venösen Schenkel mittels eines zweiten Sensors 39, beispielsweise einem optischen Sensor, erkannt, sofern es denn dort (bereits) vorliegt. Der Nachweis von Blut mittels des zweiten Sensors 39 kann rein exemplarisch mittels Infrarot-Transmissionsmessung erfolgen.

Der zweite Sensor 39 kann in bestimmten erfindungsgemäßen Ausführungsformen mit einer Einrichtung zum Erkennen von Luftblasen verbunden sein oder in einer gemeinsamen Baugruppe vorliegen. Er ist in manchen erfindungsgemäßen Ausführungsformen stromaufwärts der venösen Blutschlauchklemme 31 angeordnet. In gewissen erfindungsgemäßen Ausführungsformen ist er stromabwärts der venösen Kammer 26 bzw. der venösen Blasenkammer angeordnet.

Aus dem konstanten Blutfluss Q_B (ml/min) und der Zeitdifferenz zwischen dem ersten Zeitpunkt t1 (sec) und dem zweiten Zeitpunkt t2 (sec) kann das Volumen *V_Set* des gesamten extrakorporalen Blutkreislaufs 1000 wie folgt ermittelt werden:

$$(1) \quad V\_Set = (t2 - t1) \; x \; Q\_B \; / \; 60 \; \text{(in ml)}$$

Aufgrund der bekannten Beziehung

$$(2) \quad V\_Set = V\_Dial + V\_Schlauch$$

, mit *V_Set* für das Volumen des gesamten Blutschlauchsatzes oder -sets, kann falls das Schlauchvolumen V_*Schlauch* bekannt ist, das Volumen *V_Dial* des Dialysators berechnet werden nach

$$(3) \quad V\_Dial = V\_Set - V\_Schlauch.$$

Bauteile, die im extrakorporalen Blutkreislauf der Fig. 1 dargestellt sind, kann auch der extrakorporale Blutkreislauf der Fig. 3 aufweisen, und umgekehrt.

**Bezugszeichenliste**

| **Bezugszeichen** | **Bezeichnung** |
|---|---|
| 1000 | Extrakorporaler Blutkreislauf |
| 2000 | Dialysatkreislauf |
| 3000 | Behandlungsvorrichtung |
| 100 | Medizinische Funktionseinrichtung |
| 200 | Behandlungseinrichtung |
| 11 | Blutpumpe |
| 15 | arterieller Luftblasendetektor |
| 17 | Substituatpumpe |
| 25 | venöser Luftblasendetektor |
| 26 | venöse Kammer |
| 27 | Steuer- und/oder Regelungseinrichtung |
| 29 | arterielle Blutschlauchklemme |
| 31 | venöse Blutschlauchklemme |
| 35 | Patient |
| 37 | optischer Sensor |
| 39 | optischer Sensor |

(fortgesetzt)

| Bezugzeichen | Bezeichnung |
|---|---|
| P | Druckmesseinrichtungen |

**Patentansprüche**

**1.** Verfahren zum Ermitteln eines Füllvolumens eines extrakorporalen Blutkreislaufs (1000) oder einer Komponente hiervon mit den Schritten:

- Befüllen eines extrakorporalen Blutkreislaufs (1000) durch Einbringen eines Fluids; und
- Erfassen, Ermitteln oder Berechnen der Größe eines Volumens des eingebrachten Fluids, welches zum Befüllen des extrakorporalen Blutkreislaufs (1000) oder eines definierten oder vorbestimmten Abschnitts hiervon erforderlich ist, **dadurch gekennzeichnet, dass** ein Mindestspülvolumen (V_min) zum Spülen des extrakorporalen Blutkreislaufs (1000) zur Vorbereitung des Behandlungsverfahrens mittels der folgenden mathematischen Funktion festgelegt wird:

$$V\ min = a*V\ Set + b*V\ Dial$$

wobei:

- V_min das Mindestspülvolumen bei der Vorbereitung des Behandlungsverfahrens ist;
- V Set das Volumen der medizinischen Funktionseinrichtung (100), insbesondere eines Blutschlauchsatzes oder Blutschlauchsets, ist;
- V_Dial das Volumen der Behandlungseinrichtung (200), insbesondere eines Dialysators, ist; und
- a und b Konstanten sind.

**2.** Verfahren nach Anspruch 1, wobei a eine Zahl eines Bereichs von 1 bis 3 ist.

**3.** Verfahren nach Anspruch 1 oder 2, wobei b eine Zahl eines Bereichs von 2 bis 5 ist.

**4.** Steuer- oder Regelungseinrichtung (27), konfiguriert zur Durchführung eines Verfahrens gemäß einem der Ansprüche 1 bis 3.

**5.** Behandlungsvorrichtung, als Blutbehandlungsvorrichtung, Dialysevorrichtung, Hämodialysevorrichtung, Hämofiltrationsvorrichtung, oder Hämodiafiltrationsvorrichtung ausgestaltet, konfiguriert zur Durchführung eines Verfahrens gemäß einem der Ansprüche 1 bis 3.

**6.** Behandlungsvorrichtung nach Anspruch 5, welche eine Steuer- oder Regelungseinrichtung (27) gemäß Anspruch 4 aufweist.

**7.** Digitales Speichermedium, insbesondere in Form einer Diskette, CD oder DVD oder EPROM, mit elektrisch auslesbaren Steuersignalen, konfiguriert, um derart mit einem programmierbaren Computersystem zusammenzuwirken, dass die maschinellen Schritte eines erfindungsgemäßen Verfahrens gemäß einem der Ansprüche 1 bis 3 veranlasst werden.

**8.** Computerprogramm-Produkt mit einem auf einem maschinenlesbaren Träger gespeicherten Programmcode zur Veranlassung der maschinellen Schritte eines erfindungsgemäßen Verfahrens gemäß einem der Ansprüche 1 bis 3, wenn das Computerprogramm-Produkt auf einem Computer abläuft.

**Claims**

**1.** A method for determining a filling volume of an extracorporeal blood circuit (1000) or of a component thereof comprising the steps of:

- filling of an extracorporeal blood circuit (1000) by the introduction of a fluid; and
- detecting, determining or calculating the size of a volume of the introduced fluid, which is required for filling the extracorporeal blood circuit (1000) or a defined or predetermined section thereof, **characterized in that** a minimum flush volume (V_min) for flushing the extracorporeal blood circuit (1000) for the preparation of the treatment method is defined by means of the following mathematical function:

$$V\_min = \mathbf{a}*V\_Set + \mathbf{b}*V\_Dial$$

wherein:

- V_min is the minimum flush volume when preparing the treatment method;
- V_Set is the volume of the medical functional device (100), in particular of a blood tubing kit or blood tubing set;
- V_Dial is the volume of the treatment device (200), in particular of a dialyzer, and
- **a** and **b** are constants.

2. A method according to claim 1, wherein **a** is a number of a range of from 1 to 3.

3. A method according to claim 1 or 2, wherein **b** is a number of a range of from 2 to 5.

4. A control device or a regulating device (27), configured for executing a method according to any of claims 1 to 3.

5. A treatment apparatus, designed as a blood treatment apparatus, a dialysis apparatus, a haemodialysis apparatus, a haemofiltration apparatus or a haemodiafiltration apparatus configured for executing a method according to any of claims 1 to 3.

6. A treatment apparatus according to claim 5 comprising a control device or a regulating device (27) according to claim 4.

7. A digital storage medium, in particular in form of a disk, CD or DVD or EPROM, having electrically readable control signals, configured to interact with a programmable computer system, such that the mechanical steps of a method according to the present invention according to anyone of claims 1 to 3 are prompted.

8. A computer program product having a program code stored on a machine-readable medium for prompting the mechanical steps of the method according to the present invention according to anyone of claims 1 to 3, when the computer program product runs on a computer.

**Revendications**

1. Un procédé de détermination d'un volume de remplissage d'un circuit sanguin extracorporel (1000) ou d'un composant de celui-ci selon les étapes suivantes:

- remplissage d'un circuit sanguin extracorporel (1000) par introduction d'un fluide; et
- détection, détermination ou calcul de la grandeur du volume du fluide introduit nécessaire au remplissage du circuit sanguin extracorporel (1000) ou d'une portion définie ou prédéterminée de celui-ci, **caractérisé en ce qu'**un volume de rinçage minimum (V_min) nécessaire au rinçage du circuit sanguin extracorporel (1000) est fixé pour la préparation du procédé de traitement au moyen de la fonction mathématique suivante:

$$V\_min = \mathbf{a}*V\_Set + \mathbf{b}*V\_Dial$$

où:

- V_min représente le volume de rinçage minimum lors de la préparation du procédé de traitement;
- V_Set représente le volume du dispositif de fonctionnement médical (100), en particulier d'un assortiment de tuyaux pour le sang ou d'un set de tuyaux pour le sang;
- V_Dial représente le volume du dispositif de traitement (200), en particulier d'un dialyseur; et
- **a** et **b** sont des constantes.

2. Un procédé selon la première revendication, où **a** représente un nombre d'un intervalle de 1 à 3.

3. Un procédé selon la première ou seconde revendication, où **b** représente un nombre d'un intervalle de 2 à 5.

4. Un moyen de commande ou de régulation (27), conçu pour l'exécution d'un procédé selon l'une des revendications 1 à 3.

5. Un appareil de traitement, conçu comme un appareil de traitement du sang, un appareil de dialyse, un appareil d'hémodialyse, un appareil d'hémofiltration ou d'un appareil d'hémodiafiltration, configuré pour l'exécution d'un procédé selon l'une des revendications 1 à 3.

6. Un appareil de traitement selon la revendication 5 comprenant un moyen de commande ou de régulation (27) conformément à la revendication 4.

7. Un support de stockage numérique, en particulier sous forme de disquette, CD ou DVD ou EPROM, avec des signaux de contrôle lisibles électriquement, conçu de façon à interagir avec un système informatique programmable de telle sorte que les étapes mécaniques d'un procédé de ladite invention selon l'une des revendications 1 à 3 soient mises en oeuvre.

8. Un produit de programme informatique avec un code de programme stocké sur un support lisible par une machine pour la mise en oeuvre des étapes mécaniques d'un procédé de ladite invention conformément à l'une des revendications 1 à 3, lorsque le produit de programme informatique est exécuté sur un ordinateur.

2000 ~

1000

17

200

27

26

100

11

15

25

29

31

A

3000

*Fig. 1*

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008045422 A1
- EP 1748292 A1

- WO 2004057313 A1